(19) **Europäisches Patentamt**
**European Patent Office**
**Office européen des brevets**

(11) **EP 1 772 155 A1**

(12) # EUROPEAN PATENT APPLICATION

(43) Date of publication:
**11.04.2007 Bulletin 2007/15**

(21) Application number: **05111160.7**

(22) Date of filing: **23.11.2005**

(51) Int Cl.:
*A61K 38/21* (2006.01)      *A61K 31/4196* (2006.01)
*A61K 31/24* (2006.01)       *A61K 48/00* (2006.01)
*A61P 31/16* (2006.01)

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(30) Priority: **06.10.2005 US 244107**

(71) Applicant: **Hemispherx Biopharma**
**Philadelphia, PA 19103 (US)**

(72) Inventors:
• **Carter, William**
**19010, Philadelphia (US)**
• **Strayer, David**
**191103, Philadelphia (US)**

(74) Representative: **Spadaro, Marco et al**
**Via Calcutta, 45**
**00144 Roma (IT)**

(54) **Medicament for treating avian influenza**

(57) Avian influenza is treated with natural human alpha interferon, neuraminidase inhibitor(s) and ribavirin. Effects of influenza virus are mitigated with a dsRNA in combination with a neuraminidase influenza virus inhibitor. These two products, dsRNA, and alpha interferon, have therapeutic utility either given preventively (prophylactically) or in treatment of active disease. These unique immunological/antiviral actions, operating through immunological "cascades" ameliorates the lethal effects of viral mutation which, by causing resistance to commonly available drugs, greatly accelerates the death rate.

Figure 1

EP 1 772 155 A1

Printed by Jouve, 75001 PARIS (FR)

**Description**

The present invention relates to the use of α-interferon composed of a mixture of naturally occurring α-interferons or a synthetic, specifically configured, double-stranded ribonucleic acid (dsRNA) or both an α-interferon and a dsRNA, and optionally other components for the preparation of a medicament for combating the effects of avian influenza (AVI).

Brief description of the drawings

Figure 1 is a graph showing percent of control of influenza virus infected cells by Ampligen® as in Example 2, and

Figure 2 is a graph showing percent of control of influenza virus infected cells by Oseltamivir as in Example 2.

Description of the invention

Described is the coordinated use of both (1) an α-interferon, preferably a natural, multi-species α-interferon and conjointly therewith (2) a dsRNA for the preparation of a medicament for the treatment of the symptoms associated with AVI in patients, including human patients, infected the AVI. Procedures for attaining a favorable therapeutic and clinical result and compositions for accomplishing the same are described. Preferably the dsRNA is administered with the α-interferon and preferably the dsRNA is $rI_n \cdot r(C_{12}U)_n$, Poly A · Poly U or $rI_n \cdot r(C_{29},G)_n$, in which r is ribo.

In the context of the present invention, what is meant by "coordinated" use is, independently, either (i) co-administration, i.e. substantially simultaneous or sequential administration of the α-interferon and of the dsRNA, or (ii) the administration of a composition comprising the α-interferon and the dsRNA in combination and in a mixture, in addition to optional pharmaceutically acceptable excipients and/or vehicles.

For internal administration the α-interferon may, for example, be formulated in conventional manner for oral or rectal administration. Formulations for oral administration include aqueous solutions, syrups, elixirs, powders, granules, tablets and capsules which typically contain conventional excipients such as binding agents, fillers, lubricants, disintegrants, wetting agents, suspending agents, emulsifying agents, preservatives, buffer salts, flavoring, coloring and/or sweetening agents.

The α-interferon component of the therapeutic procedures is preferably Alferon N Injection® the only approved natural, multi-species, α-interferon available in the United States. It is the first natural source, multi-species interferon and is a consistent mixture of at least seven species of α-interferon. In contrast, the other available α-interferons are single molecular species of α-interferon made in bacteria using DNA recombinant technology. These single molecular species of α-interferon also lack an important structural carbohydrate component because this glycosylation step is not performed during the bacterial process.

Unlike species of α-interferon produced by recombinant techniques, Alferon N Injection® is produced by human white blood cells which are able to glycosylate the multiple α-interferon species. Reverse Phase HPLC studies show that Alferon N Injection® is a consistent mixture of at least seven species of alpha interferon (α2, α4, α7, α8, α10, α16, α17). This natural-source interferon has unique anti-viral properties distinguishing it from genetically engineered interferons. The high purity of Alferon N Injection® and its advantage as a natural mixture of seven interferon species, some of which, like species 8b, have greater antiviral activities than other species, for example, species 2b, which is the only component of Intron A. The superior antiviral activities for example in the treatment of chronic hepatitis C virus (HCV) and (HIV) and tolerability of Alferon N Injection® compared to other available recombinant interferons, such as Intron A and Roferon A, have been reported.

The invention includes methods of enhancing therapy against AVI or a susceptible viral infection other than AVI which has a common mechanism of viral multiplication or pathogenesis, in whole or in part, similar to that of AVI by administering to patients interferons, particularly natural human alpha interferon and together or conjointly a synthetic, specifically configured, double-stranded ribonucleic acid (dsRNA). The dsRNA of choice is Ampligen®, a synthetic, specifically configured, double-stranded ribonucleic acid (dsRNA) which retains the immunostimulatory and antiviral properties of other double-stranded RNA molecules (dsRNA) but exhibits greatly reduced toxicity. Like other dsRNA, Ampligen® can elicit the induction of interferon and other cytokines. Ampligen® has the ability to stimulate a variety of dsRNA-dependent intracellular antiviral defense mechanisms including the 2', 5'-oligoadenylate synthetase/RNase L and protein kinase enzyme pathways.

The mismatched dsRNA may be of the general formula $rI_n \cdot r(C_{12}U)_n$. In this and the other formulae that follow r = ribo. Other mismatched dsRNAs for use in the present invention are based on copolynucleotides selected from poly $(C_m,U)$ and poly $(C_mG)$ in which m is an integer having a value of from 4 to 29 and are mismatched analogs of complexes of polyriboinosinic and polyribocytidilic acids, formed by modifying $rI_n \cdot rC_n$ to incorporate unpaired bases (uracil or guanine) along the polyribocytidylate ($rC_m$) strand. Alternatively, the dsRNA may be derived from r(I) · r(C) dsRNA by modifying the ribosyl backbone of polyriboinosinic acid ($rI_n$), e.g., by including 2'-O-methyl ribosyl residues. The mismatched may be complexed with an RNA-stabilizing polymer such as lysine cellulose. Of these mismatched analogs of $rI_n · rC_n$, the preferred ones are of the general formula $rI_n · r(C_{11-14},U)_n$. or $rI_n · r(C_{29},G)_n$, and are described by Carter and Ts'o in U.S. Patent Nos. 4,130,641 and 4,024,222. The dsRNA's described therein generally are suitable for use according to the present invention.

Other examples of mismatched dsRNA for use in the invention include:

$$r (I) \cdot r (C_4, U)$$

$$r (I) \cdot r (C_7, U)$$

$$r (I) \cdot r (C_{13}, U)$$

$$r (I) \cdot r (C_{22}, U)$$

$$r (I) \cdot r (C_{20}, G)$$

and

$$r (I) \; r (C_{p\,23}, G_{>p}).$$

Alternatively the dsRNA may be the matched form, thus polyadenylic acid complexed with polyuridylic acid (poly A · poly U) may also be used.

α-interferon and/or the dsRNA may be administered for therapy by any suitable route including oral, rectal, nasal, topical (including transdermal, buccal and sublingual), vaginal and parenteral (including subcutaneous, intramuscular, intravenous intradermal, and intravitreal). It will be appreciated that the preferred route will vary with the condition and age of the recipient, the nature of the infection and the chosen active ingredient.

It is also reported the activity of interferon can potentially be amplified by the addition of a double-stranded RNA drug, Ampligen®. While interferon up-regulates certain intracellular antiviral pathways, dsRNAs, like Ampligen®, are required to fully activate these important antiviral pathways. When interferons are combined with Ampligen® synergistic antiviral and antitumor effects are seen. Moreover, Ampligen® has already shown strong antiviral activity in two separate animal models of the coronavirus (MHV-3).

In addition, as a further attribute of the dsRNA arm of the disclosed therapeutic combination therapy, synergistic antiviral and antitumor effects have been demonstrated using Ampligen® treatment in combination with all three types of interferon (α, β and γ). These synergistic effects have been seen against HIV and a variety of different histologic tumor types.

Four human tumor cell lines were studied for their response to antiproliferative effects of Ampligen® in combination with various interferons. Results indicate that (1) Ampligen® worked synergistically with all interferons in all cell lines studied; (2) growth inhibition of cells resistant to interferons can be potentiated by low doses of Ampligen®; (3) the antiproliferative effect of interferons can be potentiated by Ampligen® in Ampligen®-resistant cells; and (4) Ampligen® works by a mechanism(s) other than, or in addition to, the induction of interferon. See Montefiori, AIDS Res. and Human Retroviruses 5:193-203, 1989 and Hubbell, Int. J. Cancer 37:359-365, 1986.

The recommended dosage of the components will depend on the clinical status of the patient and the experience of the clinician in treating similar infection. As a general guideline dosage of Alferon N Injection® utilized for systemic infections is 5 to 10 million units (subcutaneous) thrice weekly. Experience to date is with dosages above 3 IU/lb of patient body weight. Oral α-interferon (Alferon LDO) has been administered as a liquid solution in the range of 500-2000 IU/day and calculated on the basis of a 150 pound human this is 3.3 to 13.3 IU/lb per day.

Our experience indicates beneficial results are obtained at dosage levels of α-interferon in excess of 450 IU, that is greater than 3 IU/pound body weight. These amounts are in contrast to and greater than Cummins use in U.S.

5,910,304 of alpha interferon administration to the pharyngeal mucosa orally or as lozenge or tablet.

The Ampligen® dose schedule is 400 mg by IV infusion twice weekly, although these amounts and/or dosage frequency may be varied by the clinician in response to the patient's condition. The components may be administered at the same time, for instance as mixture of the α-interferon and dsRNA, independently as the α-interferon then the dsRNA or the α-interferon and the dsRNA may be administered in a time-spaced manner.

Another aspect of the invention is the treatment of acute and chronic viral infections including, for example, avian influenza employing a synergistic combination of an α-interferon such as Alferon with another antiviral such as ribavirin and, optionally, also, with a dsRNA.

Interferons, particularly α-interferons and dsRNAs are discussed above.

Synergistic activity of α-interferon and ribavirin has been demonstrated to inhibit an avian virus (DucklMN/ 1525/81) in cell culture as illustrated in Example 1. The particular cells used in this study lack receptors for dsRNAs, however based upon our experience it is expected that cells with receptors for dsRNAs will also exhibit beneficial even synergistic effects of a tri-component administration of an α-interferon, an antiviral such as ribavirin, and a dsRNA such as Ampligen®. It is expected this 3-part combination will be effective in treating various acute or chronic viral infections such as HIV and severe acute respiratory syndrome as well as avian influenza.

A further aspect of the invention is conferring resistance to, or the treatment of or mitigation of the effects influenza viruses generally and avian flu virus in particular or a susceptible viral infection other than avian flu which has a common mechanism of viral multiplication or pathogenesis, in whole or in part, similar to that of avian flu using a combination of (1) an antiviral agent that inhibits influenza virus neuraminidase and possibly altering of virus particle aggregation and release, and (2) a dsRNA as described above.

As an example of antiviral agent (1) oseltamivir or its pharmaceutically acceptable salts such as the carboxylate or the phosphate which is available as TAMIFLU® from Roche as 75mg capsules for oral use. TAMIFLU® is used for postexposure and preseasonal prophylaxis of influenza infections.

The following examples further illustrate the invention.

Example 1

Tables 1 and 2 show the results of two experiments testing Alferon N in combination with ribavirin against an influenza A (H5N1) virus in Vero cells. The testing procedure involved an 18-hour pre-incubation of compounds (half-log increments) with the cells prior to virus exposure in order to activate them to an interferon-induced state. Fresh Alferon N, ribavirin, or the both used together was added again at the time of infection. After three days of incubation of virus compounds, visual examination was used to assess extent of cell destruction (cytopathic effect (CPE)) caused by the virus.

Tables 1 and 2 show actual percentages of cell destruction observed at each drug combination. The cytopathic effect (CPE) inhibition assay results indicated that Alferon N alone had a weak effect at 1,000, 3,200 and 10,000 units/ml. The combination of Alferon N plus ribavirin (32 ug/ml) showed CPE suppression that was greater than either drug alone. Ribavirin at 100 ug/ml showed a 25-50% CPE. Less CPE (i.e. protection against the virus) was observed with ribavirin (100 ug/ml) plus Alferon N (four doses).

The overall assessment is that there was improvement in cell protection when Alferon N was combined with ribavirin.

Table 1

Effect of combination of Alferon N and ribavirin on an Influenza A/Duck/MN/1525/81 (H5N1) infection in Vero cells as determined by cytopathic effect inhibition assay.

Percent Cytopathic Effect

| Alferon N (Units/ml) | Ribavirin (μg/ml) | | | |
|---|---|---|---|---|
| | 320 | 100 | 32 | 0 |
| 10,000 | 0 | 0 | 13 | 75 |
| 3,200 | 0 | 0 | 25 | 80 |
| 1,000 | 0 | 0 | 25 | 88 |
| 320 | 0 | 13 | 25 | 100 |
| 0 | 0 | 25 | 100 | 100 |

Table 2

Experiment 2. Effect of combination of Alferon N and ribavirin on an Influenza A/Duck/MN/1525/81 (H5N1) infection in Vero cells as determined by cytopathic effect inhibition assay.

| Alferon N (Units/ml) | Percent Cytopathic Effect | | | |
|---|---|---|---|---|
| | Ribavirin (µg/ml) | | | |
| | 320 | 100 | 32 | 0 |
| 10,000 | 0 | 0 | 50 | 71 |
| 3,200 | 0 | 13 | 67 | 79 |
| 1,000 | 0 | 25 | 88 | 100 |
| 320 | 0 | 25 | 100 | 100 |
| 0 | 25 | 50 | 100 | 100 |

Cinatl J, Morgenstern B, Bauer G, Chandra P, Rabenau H, Doerr HW. Treatment of SARS with human interferons. Lancet 2003; 362: 293-4. Barnard DL, Stowell VD, Seley KL, Hegde VR, Das SR, Rajappan VP, et al. Inhibition of measles virus replication by 5'-nor carbocyclic adenosine analogues. Antiviral Chem Chemother 2001; 12: 241-250.

Example 2

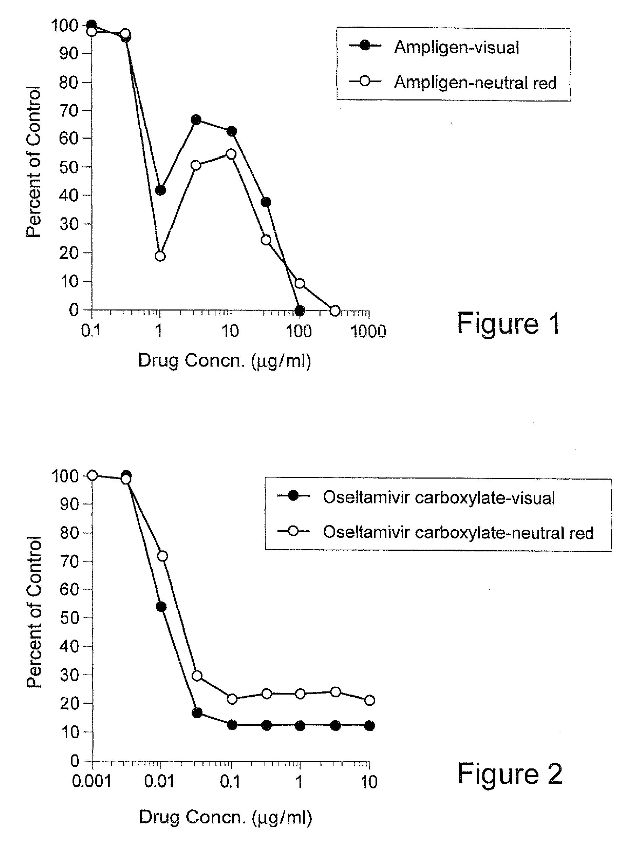

The influence of Ampligen® and oseltamivir carboxylate on influenza virus was studied to determine the cytopathic effect of these two products, individually. Ampligen® and oseltamivir carboxylate were tested against an influenza A/Duck/MN/1525/81 virus in MDCK (dog cell) cell culture. The compounds were pre-incubated with cells 18 hours prior to addition of virus. Fresh compound was applied at the time of virus addition. As shown in Figure 1, a zone of activity was observed for Ampligen® treatment between 1 and 32 µg/ml where some microwells were protected and others were not. As shown in Figure 2, oseltamivir carboxylate was active down to 0.032 µg/ml.

Example 3

In this example Ampligen® was examined in combination with oseltamivir carboxylate (Tamiflu®) for effectiveness against an influenza (avian flu) A/Duck/MN/1525/81 (H5N1) virus infection in MDCK cells. The testing procedure involved an 18-hour pre-incubation of the compounds (half-log increments) with the cells prior to virus exposure in order to activate them to an interferon-induced state. Fresh medium containing Ampligen®, oseltamivir carboxylate, or the both used together was added again at the time of infection. After three days of incubation of virus with compounds, visual and neutral red straining procedures were used to assess extent of cell destruction caused by the virus.
The data obtained and reported in the following Table 3 shows percentages of cell destruction observed at each drug combination. The cytopathic effect inhibition (CPE) assay results indicated that Ampligen® alone had a good effect only at 100 µg/ml. The combination of Ampligen® at lower concentrations plus oseltamivir carboxylate showed CPE suppression that was greater than either drug alone at several combinations.
The following table shows the effect of a combination of Ampligen® and oseltamivir carboxylate on an Influenza A/Duck/MN/1525/81 (H5N1) infection in MDCK cells as determined by cytopathic effect inhibition assay.

Table 3

| Ampligen® (µs/ml) | Percent of Cytopathic Effect | | | | | |
|---|---|---|---|---|---|---|
| | Oseltamivir Carboxylate (µg/ml) | | | | | |
| | 0.32 | 0.1 | 0.032 | 0.01 | 0.0032 | 0 |
| 100 | 0 | 0 | 0 | 0 | 0 | 0 |
| 32 | 0 | 0 | 0 | 0 | 0 | 75 |
| 10 | 0 | 0 | 0 | 58 | 63 | 100 |
| 3.2 | 13 | 13 | 29 | 100 | 71 | 100 |
| 1.0 | 13 | 13 | 50 | 100 | 100 | 100 |
| 0.32 | 13 | 13 | 50 | 100 | 100 | 100 |
| 0 | 25 | 54 | 100 | 100 | 100 | 100 |

The data was analyzed to determine a Combination Index (CI) and is based on the multiple drug-effect equation of Chou-Talalay (Chou, T.-C. and Talalay, P., J. Biol. Chem. 252: 6438-6442, 1977) in which CI values less than 1.0 indicate synergism. The results are presented in Table 4:

Table 4
CI for Experimental Values

| Ampligen® ($\mu$g/ml) | Ose_Car ($\mu$g/ml) | CI |
|---|---|---|
| 0.32 | 0.32 | 0.723 |
| 0.32 | 0.1 | 0.228 |
| 0.32 | 0.032 | 0.378 |
| 0.32 | 0.0032 | 0.160 |
| 1 | 0.032 | 0.287 |
| 1 | 0.0032 | 0.136 |
| 3.2 | 0.032 | 0.231 |
| 3.2 | 0.01 | 0.765 |
| 3.2 | 0.0032 | 0.131 |
| 10 | 0.01 | 0.312 |
| 10 | 0.0032 | 0.287 |

All combinations analyzed showed synergism indicating a clear advantage for the combination which provided an improvement in cell protection when Ampligen® was combined with and enhances oseltamivir carboxylate in inhibiting cell destruction by avian influenza.

It is expected that the combined use of Ampligen® as a prototypic dsRNA and oseltamivir as a prototypic neuraminidase inhibitor will be effective in treating various acute and chronic viral infections such as influenza, severe acute respiratory syndrome as well as avian influenza. The discovery of the combination of a neuraminidase inhibitor and dsRNA giving synergistic antiviral inhibition is an unexpected finding with great promise to mitigate the emerging problems in terms of controlling the expected pandemic with neuraminidase inhibitors given alone.

**Claims**

1. The use of alpha interferon and ribavirin, and/or neuraminidase inhibitors for the preparation of a medicament for treating avian influenza or a susceptible viral infection other than avian influenza which has a common mechanism of viral multiplication or pathogenesis, in whole or in part, similar to that of avian flu.

2. The use of a natural human alpha interferon and ribavirin and dsRNA, and a neuraminidase inhibitor for the preparation of a medicament for treating avian influenza said medicament to be administered in a coordinated manner.

3. The use according to claim 1 or 2, wherein the dsRNA is $rI_n \cdot r(C_{12}U)_n$, Poly A $\cdot$ Poly U or $rI_n \cdot r(C_{29},G)_n$, in which r is ribo.

4. The use according to claim 1 or 2 wherein the interferon is administered orally, nasally, intravenously, intramuscularly or subcutaneously in an amount of at least 3 IU per pound of the subject's body weight.

5. The use according to claim 1 or 2 in which the dsRNA is administered orally, nasally, intravenously, intramuscularly or subcutaneously.

6. The use of natural human $\alpha$-interferon in combination with ribavirin, or other chemotherapeutics such as newer neuraminidase inhibitors for the preparation of a medicament for mitigating the effects of or conferring resistance to avian influenza or a susceptible viral infection other than avian influenza which has a common mechanism of viral multiplication or pathogenesis, in whole or in part, similar to that of avian influenza comprising, prior to exposure, to the avian influenza or shortly after exposure to the avian influenza, but prior to the development of symptoms.

7. The use of natural human $\alpha$-interferon in combination with ribavirin and a dsRNA for the preparation of a medicament for mitigating the effects of or conferring resistance to avian influenza or a susceptible viral infection other than avian influenza which has a common mechanism of viral multiplication or pathogenesis, in whole or in part, similar to that of avian influenza comprising, prior to exposure, to the avian influenza or shortly after exposure to the avian influenza, but prior to the development of symptoms.

8. The use according to claim 6 or 7 wherein the interferon is administered orally, nasally, intravenously, intramuscularly or subcutaneously in an amount of at least 3 IU per pound of the subject's body weight.

9. The use according to claim 6 or 7 in which the dsRNA is administered orally, nasally, intravenously, intramuscularly or subcutaneously.

10. The use of a dsRNA in combination with a neuraminidase influenza virus inhibitor for the preparation of a medicament for mitigating the effects of or conferring resistance to influenza comprising, prior to exposure, to the influenza or shortly after exposure to the influenza, but prior to the development of symptoms.

11. The use of a dsRNA in combination with a neuraminidase influenza virus inhibitor for the preparation of a medicament for mitigating the effects of or conferring resistance to avian influenza or a susceptible viral infection other than avian influenza which has a common mechanism of viral multiplication or pathogenesis, in whole or in part, similar to that of avian influenza comprising, prior to exposure, to the avian influenza or shortly after exposure to the avian influenza, but prior to the development of symptoms, administering to a subject.

12. The use according to claim 10 or 11 wherein the dsRNA is $rI_n \cdot r(C_{12}U)_n$., Poly A $\cdot$ Poly U or $rI_n \cdot r(C_{29},G)_n$, in which r is ribo.

13. The use according to claim 10 or 11 wherein the neuraminidase inhibitor is oseltamivir, zanamivir, amantadine, rimantadine or a pharmaceutically acceptable salt thereof.

Figure 1

Figure 2

European Patent
Office

**EUROPEAN SEARCH REPORT**

Application Number

EP 05 11 1160

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | HAYDEN F G ET AL: "COMBINED INTERFERON ALPHA-2 RIMANTADINE HYDRO CHLORIDE AND RIBAVIRIN INHIBITION OF INFLUENZA VIRUS REPLICATION IN-VITRO" ANTIMICROBIAL AGENTS AND CHEMOTHERAPY, vol. 25, no. 1, 1984, pages 53-57, XP009063516 ISSN: 0066-4804 | 1,6 | A61K38/21 A61K31/4196 A61K31/24 A61K48/00 A61P31/16 |
| Y | * abstract; tables 1,2 * | 2-5,7-9 | |
| X | D'AGOSTINI C ET AL: "Combination therapy with amantadine and immunomodulators potentiates antiviral effects of influenza A virus-infected mice" ANTIVIRAL RESEARCH, vol. 20, no. SUPPL. 1, 1993, page 160, XP009063517 & SIXTH INTERNATIONAL CONFERENCE ON ANTIVIRAL RESEARCH; VENICE, ITALY; APRIL 25-30, 1993 ISSN: 0166-3542 * abstract * | 1,6 | |
| | | | **TECHNICAL FIELDS SEARCHED (IPC)** |
| X | ANONYMOUS: "Ampligen ® Enhances the Effectiveness of Tamiflu Against Avian Influenza; Second Independent Preclinical Study Confirms dsRNA Increases Flu Vaccine Effectiveness" BUSINESS WIRE, [Online] 6 September 2005 (2005-09-06), XP002372563 Philadelphia Retrieved from the Internet: URL:http://www.findarticles.com/p/articles /mi_m0EIN/is_2005_Sept_6/ai_n15343872/prin t> [retrieved on 2006-03-16] | 10-13 | A61K A61P |
| Y | * the whole document * | 2-5,7-9 | |

-/--

The present search report has been drawn up for all claims

| Place of search | Date of completion of the search | Examiner |
|---|---|---|
| Munich | 16 March 2006 | Vandenbogaerde, A |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

&  : member of the same patent family, corresponding document

EPO FORM 1503 03.82 (P04C01)

**European Patent Office**

**EUROPEAN SEARCH REPORT**

Application Number

EP 05 11 1160

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 2005/009337 A (HEMISPHERX BIOPHARMA; CARTER, WILLIAM, A; STRAYER, DAVID) 3 February 2005 (2005-02-03) * abstract *<br><br>----- | 1-13 | |
| | | | TECHNICAL FIELDS SEARCHED (IPC) |

The present search report has been drawn up for all claims

| Place of search | Date of completion of the search | Examiner |
|---|---|---|
| Munich | 16 March 2006 | Vandenbogaerde, A |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 05 11 1160

This annex lists the patent family members relating to the patent documents cited in the above-mentioned European search report.
The members are as contained in the European Patent Office EDP file on
The European Patent Office is in no way liable for these particulars which are merely given for the purpose of information.

16-03-2006

| Patent document cited in search report | Publication date | Patent family member(s) | Publication date |
|---|---|---|---|
| WO 2005009337 A | 03-02-2005 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4130641 A
- US 4024222 A
- US 5910304 A

**Non-patent literature cited in the description**

- **MONTEFIORI.** *AIDS Res. and Human Retroviruses,* 1989, vol. 5, 193-203
- **HUBBELL.** *Int. J. Cancer,* 1986, vol. 37, 359-365
- **CINATL J ; MORGENSTERN B ; BAUER G ; CHANDRA P ; RABENAU H ; DOERR HW.** Treatment of SARS with human interferons. *Lancet,* 2003, vol. 362, 293-4
- **BARNARD DL ; STOWELL VD ; SELEY KL ; HEGDE VR ; DAS SR ; RAJAPPAN VP et al.** Inhibition of measles virus replication by 5'-nor carbocyclic adenosine analogues. *Antiviral Chem Chemother,* 2001, vol. 12, 241-250
- **CHOU, T.-C. ; TALALAY, P.** *J. Biol. Chem.,* 1977, vol. 252, 6438-6442